# EUROPEAN PATENT SPECIFICATION

(11) **EP 2 839 286 B1**
(45) Date of publication and mention of the grant of the patent: **28.02.2018**
(21) Application number: 13778621.6
(22) Date of filing: 11.04.2013
(51) Int. Cl.: G01N 33/548

(54) **COMPOUNDS AND METHODS FOR PREPARATION OF CONJUGATE REAGENTS**
VERBINDUNGEN UND VERFAHREN ZUR HERSTELLUNG VON KONJUGATREAGENZIEN
COMPOSÉS ET PROCÉDÉS DE PRÉPARATION DE RÉACTIFS CONJUGUÉS

(30) Priority: 18.04.2012 US 201261625977 P
(43) Date of publication of application: 25.02.2015
(73) Proprietor: Siemens Healthcare Diagnostics Inc., Tarrytown, NY 10591 (US)
(72) Inventor: TENG, Zhu, Boothwyn, PA 19061 (US); GENG, Liping, Newark, DE 19711 (US); DRINAN, Martin, Newark, DE 19711 (US)
(74) Representative: Schweitzer, Klaus
(86) International application number: PCT/US2013/036111
(87) International publication number: WO 2013/158454

(56) References cited:
- WO-A1-94/09368
- US-A1- 2005 226 843
- US-A1- 2007 166 277
- US-B1- 7 179 660

## Description

### BACKGROUND

This disclosure relates to compounds and methods for the preparation of conjugate reagents that comprise a support bound to a specific binding pair member.

In the field of medicine and clinical chemistry, supports comprising one or more specific binding partners are used, for example, in specific binding assays, affinity purification of substances, separation of cells, enzymatic processes, and drug delivery systems.

There is a continuing need for approaches to the preparation of such conjugate reagents. For example, there is a continuing need for conjugate reagents that may be used in assays to accurately determine one or both of a presence and an amount of an analyte in samples suspected of containing the same.

### SUMMARY

The invention pertains to a composition and methods as claimed. Some examples in accordance with the principles described herein are directed to a composition comprising a support having on a surface thereof at least an inner polysaccharide layer and an outer polysaccharide layer wherein the outer polysaccharide layer has pendant moieties each comprising a terminal amine functionality. The point of linkage of the pendant moiety to the outer polysaccharide layer does not comprise an amide bond. The composition may be employed in a method of conjugating a member of a specific binding pair to a particle. The method comprising reacting the terminal amine functionality of the composition with the member of the specific binding pair, which comprises an amine-reactive functionality.

Some examples in accordance with the principles described herein are directed to a method of forming a conjugate of a hapten and a particle wherein the particle comprises an inner polysaccharide layer and an outer polysaccharide layer that comprises an aldehyde group. The method comprises forming a linkage between the hapten functionalized with an amine-reactive functionality and the aldehyde group with a linking compound comprising two terminal amine groups. One of the terminal amine groups reacts with the aldehyde group of the outer polysaccharide layer to form a non-amide bond and the other of the terminal amine groups reacts with the amine-reactive functionality of the hapten.

Some examples in accordance with the principles described herein are directed to a method of forming a conjugate of a steroid hormone and a particle wherein the particle comprises an inner aminodextran layer and an outer dextran aldehyde layer. In the method, pendant moieties each comprising a terminal amine group are formed on the particle by reacting amine groups of a linking compound, which comprises an alkylene or poly(ether alkylene) chain and two terminal amine groups, with aldehyde groups of the outer dextran aldehyde. A point of linkage of the pendant moiety to the outer dextran aldehyde layer does not comprise an amide bond linkage. The terminal amine group of the pendant moiety is reacted with the steroid hormone comprising an amine-reactive functionality to form the conjugate of the steroid hormone and the particles.

### BRIEF DESCRIPTION OF THE DRAWINGS

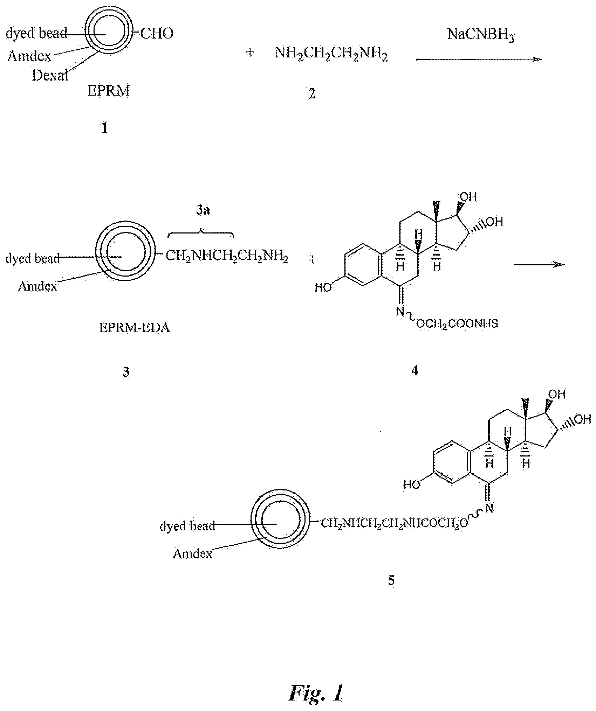

Fig. 1 is a depiction of an example of a reaction scheme for preparing compounds in accordance with the principles described herein.

### DETAILED DESCRIPTION OF SPECIFIC EMBODIMENTS

In some examples in accordance with the principles described herein, the present inventors have discovered a universal reagent that may be employed in the preparation of conjugates of supports and members of a specific binding pair (sbp) such as, for example, haptens. The reagent is universal in that the same reagent may be employed to form individual supports comprising any one of a number of different sbp members. The universal reagent exhibits enhanced coupling efficiency during a process of linking to an sbp member. The resulting support-sbp member conjugates may be employed in an assay for an analyte. The conjugates demonstrate good binding affinity between the sbp member portion, e.g., hapten, of the conjugate and a corresponding sbp member employed in an assay such as, e.g., an antibody for the hapten, which depending on an assay format is also an antibody for the analyte.

As mentioned above, some examples in accordance with the principles described herein are directed to a composition comprising a support having on a surface thereof at least an inner polysaccharide layer and an outer polysaccharide layer wherein the outer polysaccharide layer has pendant moieties comprising a terminal amine group. The point of linkage of the pendant moiety to the outer polysaccharide layer does not comprise an amide bond. The composition may be employed in a method of conjugating a member of a specific binding pair to a particle. The method comprises reacting the terminal amine group of the composition with the member of the specific binding pair that comprises an amine-reactive functionality.

The term "support" includes any organic or inorganic, solid or fluid, water insoluble material, which may be transparent or partially transparent. The support can have any of a number of shapes including, but not limited to, particle, including bead, film, membrane, tube, well, strip, rod, planar surface such as, e.g., plate, dendrimer, for example. Depending on the type of assay, the support may or may not be suspendable in the medium in which it is employed. Examples of suspendable supports are polymeric materials such as latex, lipid bilayers or liposomes, oil droplets, cells and hydrogels. Other support compositions include polymers, such as nitrocellulose, cellulose acetate, poly (vinyl chloride), polyacrylamide, polyacrylate, polyethylene, polypropylene, poly(4-methylbutene), polystyrene, polymethacrylate, poly(ethylene terephthalate), nylon, poly(vinyl butyrate), for example; either used by themselves or in conjunction with other materials. Other supports include, but are not limited to, polysaccharides, particularly crosslinked polysaccharides, such as agarose, dextran, cellulose, and starch, for example.

When the support is a particle, the average diameter of the particle is at least about 0.02 microns and not more than about 100 microns. In some examples, the particles have an average diameter from about 0.05 microns to about 20 microns, or from about 0.3 microns to about 10 microns, for example. The particle may be organic or inorganic, magnetic or non-magnetic, swellable or non-swellable, porous or non-porous, preferably of a density approximating water, generally from about 0.7 g/mL to about 1.5 g/mL, and composed of material that can be transparent, partially transparent, or opaque. The particles can be biological materials such as cells and microorganisms, e.g., erythrocytes, leukocytes, lymphocytes, hybridomas, streptococcus, Staphylococcus aureus, E. coli, viruses, and the like. The particles can also be particles comprised of organic and inorganic polymers, liposomes, latex particles, phospholipid vesicles, chylomicrons, lipoproteins, and the like. In some examples, the particles are latex particles.

The term "polysaccharide" means a carbohydrate containing three or more non-modified or modified monosaccharide units, such as, for example, dextran including aminodextran, dextran aldehyde and carboxydextran, for example, starch, glycogen, inulin, levan, mannan, agarose, galactan, and polyribose. The polysaccharide can be hydrolyzed into simpler monosaccharide units. The term "dextran" means a polysaccharide consisting of linear 1-carbon to 6-carbon linked (98%) glucose units; a polymerized glucose. The term "aminodextran" means a dextran having multiple amine functionalities per dextran molecule. The phrase "dextran aldehyde" means a dextran having multiple aldehyde functionalities per dextran molecule.

The phrases "inner polysaccharide layer" and "outer polysaccharide layer" refer to the relative position of one layer of polysaccharide with respect to another layer of polysaccharide on a surface of a support. The inner polysaccharide layer is disposed on the surface of a support inside the outer polysaccharide layer although the outer polysaccharide layer may or may not be directly disposed over the inner polysaccharide layer.

The phrase "pendant moieties comprising a terminal amine group" refers to moieties that comprise a linear chain of atoms wherein a terminal carbon atom of the chain comprises an amine group. The chain of atoms, not counting hydrogen atoms, may be from about 2 to about 100 atoms, or 2 to about 75 atoms, or about 2 to about 50 atoms, or about 2 to about 40 atoms, or about 2 to about 30 atoms, or about 2 to about 20 atoms, or about 2 to about 15 atoms, or about 2 to about 10 atoms, or about 3 to about 50 atoms, or 3 to about 40 atoms, or about 3 to about 30 atoms, or about 3 to about 20 atoms, or about 3 to about 15 atoms, or about 3 to about 10 atoms, or about 4 to about 30 atoms, or about 4 to about 20 atoms, for example, each independently selected from the group consisting of carbon, oxygen, sulfur, nitrogen, and phosphorous. The number of heteroatoms in such pendant moieties is dependent on the size of the linking compound and, in some examples, the number is in the range of from 0 to about 30, or 1 to about 25, or about 2 to about 20, or about 2 to about 15, or about 2 to about 10, or about 3 to about 10, or about 3 to about 5, for example. The heteroatoms may be in the form of one or more functionalities, such as, for example, ether, ester, amide, urea, carbamate, sulfonamide, thioether, hydrazone, hydrazide, amidine, and phosphate ester. The linear chain may include one or more branched atoms such as, for example, tertiary carbon atoms and dendrimers.

In some examples in accordance with the principles described herein, the pendant moiety comprises alkylene, which refers to a branched or unbranched saturated divalent hydrocarbon radical that may comprise a chain of carbon atoms of about 2 to about 30, or about 2 to about 25, or about 2 to about 20, or about 2 to about 15, or about 2 to about 10, about 2 to about 5. In some examples the alkylene is, but is not limited to, methylene, ethylene, propylene, 2-methylpropylene, 1,2-dimethylpropylene, butylene, pentylene, hexylene, octylene, nonylene, decylene, for example. In accordance with the principles described herein, the alkylene pendant moiety comprises a terminal amine group.

In some examples in accordance with the principles described herein, the pendant moiety comprises poly(alkylene ether), which refers to alternating ether linkages and alkylene moieties. The number of ether linkages and alkylene moieties may be about 2 to about 50, or about 2 to about 40, or about 2 to about 30, or about 2 to about 25, or about 2 to about 20, or about 2 to about 15, or about 2 to about 10, about 2 to about 5. In some examples the alkylene of the poly(alkylene ether) is, but is not limited to, methylene, ethylene, propylene, 2-methylpropylene, 1,2-dimethylpropylene, butylene, pentylene, hexylene, octylene, nonylene, decylene, for example. In some examples the pendant moiety may be represented by the formula: -(CH₂CH₂O)ₘ- or -CH₂(CH₂CH₂O)ₘ- or -(CH₂CH₂O)ₘ-CH₂- wherein m is about 2 to about 30, or about 2 to about 25, or about 2 to about 20, or about 2 to about 15, or about 2 to about 10, about 2 to about 5, or about 2 to about 4, or about 2 to about 3, or about 3 to about 5, or about 3 to about 4, or about 4 to about 5. In accordance with the principles described herein, the poly(alkylene ether) pendant moiety comprises a terminal amine group.

The point of linkage of the pendant moiety to the outer polysaccharide layer does not comprise an amide bond. This means that the linkage of the pendant moiety to the outer polysaccharide layer does not comprise a functional group such as, for example, -C(O)NH- or -NHC(O)-. The phrase "point of linkage" refers to the point where a functionality of the outer polysaccharide layer has reacted with a corresponding functionality of the pendant moiety during the attachment of the pendant moiety to the outer polysaccharide. For example, the point of linkage for an outer polysaccharide that comprises aldehyde groups would be at the aldehyde groups.

Sbp members include, but are not limited to, antibodies, antibody fragments, receptors, oligonucleotides, polynucleotides, nucleotide-binding proteins, lectins, haptens, antigens, immunoglobulin binding proteins, avidin, streptavidin, and biotin, for example. In some examples the sbp member is a hapten, which is a compound capable of binding specifically to corresponding antibodies, but does not itself act as an immunogen (or antigen) for preparation of the antibodies. Antibodies that recognize a hapten can be prepared against compounds comprised of the hapten linked to an immunogenic (or antigenic) carrier. In some examples, the hapten is a hydrophobic hapten, which is a hapten that is more soluble in a hydrophobic medium than in a hydrophilic medium. The hydrophobic hapten is fat soluble, which means that the hydrophobic hapten is soluble in one or more of fats, oils, and lipids, for example. In some examples, the hydrophobic haptens include, but are not limited to, steroid hormones, for example. Steroid hormones include, by way of illustration and not limitation, progestogens, estrogens, androgens, glucosteroids, mineralocorticoids, and secosteroids, for example. In some examples in accordance with the principles described herein, the hydrophobic hapten is progesterone, testosterone, estriol, estradiol, cortisol, vitamin D, or vitamin B12, for example.

As mentioned above, some examples in accordance with the principles described herein are directed to methods of forming a conjugate of a hapten, which may be a hydrophobic hapten, and a particle wherein the particle comprises an inner polysaccharide layer and an outer polysaccharide layer that comprises an aldehyde group. The method comprises forming a linkage between the hapten functionalized with an amine-reactive functionality and the aldehyde group of the outer polysaccharide layer using a linking compound comprising two terminal amine groups. One of the terminal amine groups reacts with the aldehyde group to form a non-amide functionality and the other of the terminal amine groups reacts with the amine-reactive functionality. The linking compound comprises an alkylene chain or a poly(alkylene ether) chain where two amine groups are at the terminus of the chain and, thus, the linking compound may be referred to as a terminal diamine compound.

"Amine reactive functional group" means a functionality reactive with an amine group, usually by virtue of nucleophilicity or basicity of the amine, such as, but not limited to, an aldehyde; a functionalized aldehyde such as, e.g., a carboxymethoxylamine-functionalized aldehyde; a ketone; a functionalized ketone such as, e.g., an a-keto carboxylic acid; an epoxy group; a functionalized alcohol such as, e.g., a tosylate; for example.

In one example in accordance with the principles described herein, the above method comprises reacting the aldehyde group of the outer polysaccharide layer with one of the two terminal amine groups of the linking compound to form a particle with a pendant moiety comprising a terminal amine group, which is the other of the terminal amine groups of the linking compound. The terminal amine group of the pendant moiety is reacted with the amine-reactive functionality of the hapten to form the conjugate.

In another example in accordance with the principles described herein, the above method comprises reacting one of the two terminal amine groups of the linking compound with the amine-reactive functionality of the hapten to form a hapten with a moiety comprising a terminal amine group, which is the other of the two terminal amine groups of the linking compound. The conjugate is formed by reacting the aldehyde group of the outer polysaccharide layer with the terminal amine group of the moiety that was added to the hapten.

Some examples in accordance with the principles described herein are directed to a method of forming a conjugate of a steroid hormone and a particle wherein the particle comprises an inner aminodextran layer and an outer dextran aldehyde layer. The method comprises forming pendant moieties on the particle by reacting aldehyde groups of the outer dextran aldehyde with an amine group of a linking compound that comprises an alkylene or poly(alkylene ether) chain and terminal amine groups at each end of the chain. A point of linkage of the pendant moiety to the outer dextran aldehyde layer does not comprise an amide bond. Each of the pendant moieties comprises a terminal amine group, which is reacted with the steroid hormone comprising an amine-reactive functionality to form the conjugate of the steroid hormone and the particle.

In some examples in accordance with the principles described herein, the particles comprise a label, which is any molecule that produces or can be induced to produce a signal, and may be, by way of illustration and not limitation, a chemiluminescent compound (chemiluminescer), a sensitizer, a fluorescent compound (fluorescer), a radiolabel, or an enzyme, for example.

The label can directly produce a signal and, therefore, additional components are not required to produce a signal. Numerous organic molecules, for example fluorescers, are able to absorb ultraviolet and visible light, where the light absorption transfers energy to these molecules and elevates them to an excited energy state. This absorbed energy is then dissipated by emission of light at a second wavelength. Other labels that directly produce a signal include radioactive isotopes and dyes.

Alternately, the label may need other components to produce a signal. The label along with such other components comprise a signal producing system (sps), which would include all the components required to produce a measurable signal. Such other components include, but are not limited to, substrates, coenzymes, quenchers, enhancers, additional enzymes, substances that react with enzymatic products, catalysts, activators, cofactors, inhibitors, scavengers, metal ions, and a specific binding substance required for binding of signal generating substances.

Suitable labels include, by way of illustration and not limitation, dyes; fluorescers, such as fluorescein, rhodamine compounds, phycoerythrin, phycocyanin, allophycocyanin, o-phthaldehyde, fluorescent rare earth chelates, and fluorescamine; chemiluminescers such as isoluminol, and acridinium compounds; sensitizers, such as eosine, 9,10-dibromoanthracene, methylene blue, hematoporphyrin, phthalocyanines, chlorophyll, rose bengal; for example. "Signal producing system", as used herein, means one or more components, at least one component being a detectable label, which generates a detectable signal that relates to the amount of bound and/or unbound label, i.e. the amount of label bound or not bound to the compound being detected. The label may be associated to the support by direct or indirect bonding, adsorption, absorption, incorporation, or solution, for example.

In some examples in accordance with the principles described herein, the support is a latex particle. In some examples, the latex particle comprises a label that is incorporated in the latex particle. In some examples, the label is a chemiluminescent composition or a sensitizer composition, for example. In some examples, the hydrophobic hapten of the reagent conjugate is selected from the group consisting of progestogens, estrogens, androgens, glucosteroids, mineralocorticoids, and secosteroids, for example.

### Preparation of Reagent Conjugates

Examples of syntheses of reagents and conjugates are described with reference to Fig. 1 by way of illustration and not limitation. Other approaches may be employed to form the reagents and conjugates consistent with the principles described herein. In the example depicted in Fig. 1, a latex particle **1** (EPRM) having incorporated therein a chemiluminescent compound (dye) and having an inner aminodextran layer (Amdex) and an outer dextran aldehyde layer (Dexal) is prepared by a procedure similar to that described in U.S. Patent No. 7,179,660. Aldehyde groups on the outer dextran aldehyde layer are reacted with ethylene diamine **2** under reductive amination conditions to form reagent **3** (EPRM-EDA) having pendant moieties comprising an ethylene chain and a terminal amine group. The reductive amination conditions include the use of a reducing agent such as, for example, a metal hydride (e.g., sodium borohydride or potassium borohydride), sodium cyanoborohydride, or sodium triacetoxyborohydride. The reaction is carried out in an aqueous medium at a temperature during the reaction of about 20°C to about 100°C, or about 30°C to about 50°C, for a period of about 1 hour to about 48 hours, or about 5 hours to about 24 hours. The point of linkage **3a** of the pendant moieties with the outer dextran aldehyde layer comprises a secondary amine linkage.

Reagent **3** is reacted with carboxymethoxylamine-activated estradiol **4** to form conjugate reagent **5** having estradiol linked to latex particle **1.** The reaction is carried out by first combining carboxymethoxylamine-activated estradiol **4** with an activation agent such as, for example, N-hydroxysuccinimide (NHS) ester and 1-ethyl-3-(3-dimethylaminopropyl)carbodiimide (EDAC) in an aqueous medium at a temperature of about 15°C to about 40°C, or about 20°C to about 30°C, or at ambient temperature for a period of about 1 hour to about 5 hours, or about 1 hour to about 3 hours prior to addition of reagent 3. After addition of reagent **3**, the medium is agitated at about 15°C to about 40°C, or about 200°C to about 30°C, or at ambient temperature for a period of about 1 hour to about 48 hours, or about 5 hours to about 24 hours.

### General Description of Assays Utilizing the Present Conjugates

Examples of support-sbp member conjugates prepared in accordance with the principles described herein may be employed as a label reagent in assays for the determination of an analyte in a sample.

An assay can be performed either without separation (homogeneous) or with separation (heterogeneous) of any of the assay components or products. Heterogeneous assays usually involve one or more separation steps and can be competitive or non-competitive. Immunoassays may involve labeled or non-labeled reagents. Immunoassays involving non-labeled reagents usually comprise the formation of relatively large complexes involving one or more antibodies prepared from immunogenic conjugates in accordance with the principles described herein. Such assays include, for example, immunoprecipitin and agglutination methods and corresponding light scattering techniques such as, e.g., nephelometry and turbidimetry, for the detection of antibody complexes. Labeled immunoassays include, but are not limited to, chemiluminescence immunoassays, enzyme immunoassays, fluorescence polarization immunoassays, radioimmunoassays, inhibition assays, induced luminescence assays, and fluorescent oxygen channeling assays, for example, where a label conjugate in accordance with the principles described herein is employed in the assay.

One general group of immunoassays includes immunoassays using a limited concentration of the present conjugate reagent. Another group of immunoassays involves the use of an excess of one or more of the principal reagents such as, for example, an excess of the present conjugate reagent. Another group of immunoassays are separation-free homogeneous assays in which a labeled reagent in accordance with the principles described herein modulates the label signal upon binding of the present conjugate to an antibody in the sample.

As mentioned above, the assays can be performed either without separation (homogeneous) or with separation (heterogeneous) of any of the assay components or products. Homogeneous immunoassays are exemplified by the EMIT® assay (Siemens Healthcare Diagnostics Inc., Deerfield, IL) disclosed in Rubenstein, et al., U.S. Patent No. 3,817,837, column 3, line 6 to column 6, line 64; immunofluorescence methods such as those disclosed in Ullman, et al., U.S. Patent No. 3,996,345, column 17, line 59, to column 23, line 25; enzyme channeling immunoassays ("ECIA") such as those disclosed in Maggio, et al., U.S. Patent No. 4,233,402, column 6, line 25 to column 9, line 63; the fluorescence polarization immunoassay ("FPIA") as disclosed, for example, in, among others, U.S. Patent No. 5,354,693; and enzyme immunoassays such as the enzyme linked immunosorbant assay ("ELISA"). Exemplary of heterogeneous assays are the radioimmunoassay, disclosed in Yalow, et al., J. Clin. Invest. 39:1157 (1960). The relevant portions of the above disclosures are all incorporated herein by reference.

Other enzyme immunoassays are the enzyme modulate mediated immunoassay ("EMMIA") discussed by Ngo and Lenhoff, FEBS Lett. (1980) 116:285-288; the substrate labeled fluorescence immunoassay ("SLFIA") disclosed by Oellerich, J. Clin. Chem. Clin. Biochem. (1984) 22:895-904; the combined enzyme donor immunoassays ("CEDIA") disclosed by Khanna, et al., Clin. Chem. Acta (1989) 185:231-240; homogeneous particle labeled immunoassays such as particle enhanced turbidimetric inhibition immunoassays ("PETINIA"), particle enhanced turbidimetric immunoassay ("PETIA"), etc.; and the like.

Other assays include the sol particle immunoassay ("SPIA"), the disperse dye immunoassay ("DIA"); the metalloimmunoassay ("MIA"); the enzyme membrane immunoassays ("EMIA"); luminoimmunoassays ("LIA"); and so forth. Other types of assays include immunosensor assays involving the monitoring of the changes in the optical, acoustic and electrical properties of the present conjugate upon the binding to an antibody. Such assays include, for example, optical immunosensor assays, acoustic immunosensor assays, semiconductor immunosensor assays, electrochemical transducer immunosensor assays, potentiometric immunosensor assays, amperometric electrode assays.

Heterogeneous assays usually involve one or more separation steps and can be competitive or non-competitive. A variety of competitive and non-competitive heterogeneous assay formats are disclosed in Davalian, et al., U.S. Pat. No. 5,089,390, column 14, line 25 to column 15, line 9, incorporated herein by reference. In an example of a competitive heterogeneous assay, a support having an antibody for an analyte bound thereto is contacted with a medium containing the sample suspected of containing the analyte and a labeled conjugate in accordance with the principles described herein. Analyte in the sample competes with the present conjugate bearing the detectable label for binding to the antibody for the analyte. After separating the support and the medium, the label activity of the support or the medium is determined by conventional techniques and is related to the amount of analyte in the sample.

In some examples, a sample to be analyzed is combined in an assay medium with an antibody for the analyte and labeled conjugate in accordance with the principles described herein. The medium is examined for one or both of the presence and amount of a complex comprising analyte and the antibody for analyte where the presence and/or the amount of such complex indicates the presence and/or amount of analyte in the sample.

The sample to be analyzed is one that is suspected of containing an analyte, that is, the compound of interest. The samples are preferably from a mammalian subject, e.g., humans or other animal species and include biological fluids such as whole blood, serum, plasma, sputum, lymphatic fluid, semen, vaginal mucus, feces, urine, spinal fluid, saliva, stool, cerebral spinal fluid, tears, mucus, and the like; biological tissue such as hair, skin, sections or excised tissues from organs or other body parts; and so forth. In many instances, the sample is whole blood, plasma or serum.

The sample can be prepared in any convenient medium. Conveniently, the sample may be prepared in an assay medium, which is discussed more fully hereinbelow. In some instances a pretreatment may be applied to the sample such as, for example, to lyse blood cells. In some examples, such pretreatment is performed in a medium that does not interfere subsequently with an assay.

The assays are normally carried out in an aqueous buffered medium at a moderate pH, generally that which provides optimum assay sensitivity. The aqueous medium may be solely water or may include from 0.1 to about 40 volume percent of a cosolvent. The pH for the medium will be in the range of about 4 to about 11, or in the range of about 5 to about 10, or in the range of about 6.5 to about 9.5. The pH will usually be a compromise between optimum binding of the binding members of any specific binding pairs, the pH optimum for other reagents of the assay such as members of the signal producing system, and so forth. Various buffers may be used to achieve the desired pH and maintain the pH during the assay. Illustrative buffers include borate, phosphate, carbonate, tris, barbital, PIPES, HEPES, MES, ACES, MOPS, BICINE, and the like. The particular buffer employed is not critical, but in an individual assay one or another buffer may be preferred.

Various ancillary materials may be employed in the assay methods. For example, in addition to buffers the medium may comprise stabilizers for the medium and for the reagents employed. In some embodiments, in addition to these additives, proteins may be included, such as albumins; organic solvents such as formamide; quaternary ammonium salts; polyanions such as dextran sulfate; binding enhancers, e.g., polyalkylene glycols; polysaccharides such as dextran, trehalose, or the like. The medium may also comprise agents for preventing the formation of blood clots. Such agents are well known in the art and include, for example, EDTA, EGTA, citrate, heparin, and the like. The medium may also comprise one or more preservatives as are known in the art such as, for example, sodium azide, neomycin sulfate, PROCLIN® 300, Streptomycin, and the like. Any of the above materials, if employed, is present in a concentration or amount sufficient to achieve the desired effect or function.

Depending on the nature of the assay employed, the medium may comprise one or more components such as, for example, other members of a signal producing system of which the label is a part.

One or more incubation periods may be applied to the medium at one or more intervals including any intervals between additions of various reagents employed in an assay including those mentioned above. The medium is usually incubated at a temperature and for a time sufficient for binding of various components of the reagents and binding of analyte in the sample to occur. Moderate temperatures are normally employed for carrying out the method and usually constant temperature, preferably, room temperature, during the period of the measurement. In some examples, incubation temperatures range from about 5° to about 99°C, or from about 15°C to about 70°C, or about 20°C to about 45°C. The time period for the incubation, in some examples, is about 0.2 seconds to about 24 hours, or about 1 second to about 6 hours, or about 2 seconds to about 1 hour, or about 1 minute to about 15 minutes. The time period depends on the temperature of the medium and the rate of binding of the various reagents, which is determined by the association rate constant, the concentration, the binding constant and dissociation rate constant.

In an example of a method for determining an analyte in a sample suspected of containing the analyte, a combination is provided in a medium where the combination includes the sample, an antibody for the analyte, and a label conjugate in accordance with the principles described herein. The medium is examined for one or both of the presence and amount of a complex comprising the analyte and the antibody for analyte. The presence and/or the amount of the complex indicates the presence and/or amount of the analyte in the sample.

Some known assays utilize a signal producing system (sps) that employs first and second sps members. The designation "first" and "second" is completely arbitrary and is not meant to suggest any order or ranking among the sps members or any order of addition of the sps members in the present methods. The sps members may be related in that activation of one member of the sps produces a product such as, e.g., light, which results in activation of another member of the sps.

In some embodiments of known assays, the sps members comprise a sensitizer such as, for example, a photosensitizer, and a chemiluminescent composition where activation of the sensitizer results in a product that activates the chemiluminescent composition. The second sps member usually generates a detectable signal that relates to the amount of bound and/or unbound sps member, i.e., the amount of sps member bound or not bound to the analyte being detected or to an agent that reflects the amount of the analyte to be detected. In some examples in accordance with the principles described herein, one of either the sensitizer reagent or the chemiluminescent reagent is a conjugate reagent prepared in accordance with the principles described herein. Examples of photosensitizers and chemiluminescent reagents that may be utilized are those set forth in U.S. Pat. Nos. 5,340,716 and 6,251,581, the relevant disclosures of which are incorporated herein by reference.

In a particular example, an induced luminescence immunoassay may be employed. The induced luminescence immunoassay is referred to in U.S. Pat. No. 5,340,716 (Ullman), which disclosure is incorporated herein by reference. The assay employs a photosensitizer reagent and a chemiluminescent reagent. In one approach, the assay uses, as a photosensitizer reagent, a label particle-conjugate where the label of the particle-conjugate is a photosensitizer and where the label particle-conjugate is in accordance with the principles described herein. The chemiluminescent reagent comprises an antibody for the analyte. The analyte competes with the particle-conjugate, which comprises an analyte as the hapten of the conjugate, for the antibody for the analyte. If the analyte is present, the fewer is the number of molecules of labeled particle-conjugate that come into close proximity with the chemiluminescent compound. Therefore, there will be a decrease in the assay signal. The photosensitizer generates singlet oxygen and activates the chemiluminescent reagent when the two labels are in close proximity. The activated chemiluminescent reagent subsequently produces light. The amount of light produced is related to the amount of the complex formed, which in turn is related to the amount of analyte present in the sample.

In another particular example of an induced luminescence immunoassay, the assay uses, as a chemiluminescent reagent, a label particle-conjugate in accordance with the principles described herein where the label of the particle-conjugate is a chemiluminescent compound. The photosensitizer reagent comprises an antibody for the analyte. The analyte competes with the particle-conjugate for binding to the antibody for the analyte. If the analyte is present, the fewer is the number of molecules of labeled particle-conjugate that come into close proximity with the photosensitizer reagent. Therefore, there will be a decrease in the assay signal. The photosensitizer generates singlet oxygen and activates the chemiluminescent compound when the two labels are in close proximity. The activated chemiluminescent compound subsequently produces light. The amount of light produced is related to the amount of the complex formed, which in turn is related to the amount of analyte present in the sample.

The concentration of the analyte that may be assayed generally varies from about 10⁻⁵ to about 10⁻¹⁷ M, more usually from about 10⁻⁶ to about 10⁻¹⁴ M. Considerations, such as whether the assay is qualitative, semi-quantitative or quantitative (relative to the amount of the analyte present in the sample), the particular detection technique and the expected concentration of the analyte normally determine the concentrations of the various reagents.

The concentrations of the various reagents in the assay medium will generally be determined by the concentration range of interest of the analyte, the nature of the assay, and the like. However, the final concentration of each of the reagents is normally determined empirically to optimize the sensitivity of the assay over the range of interest. That is, a variation in concentration of analyte that is of significance should provide an accurately measurable signal difference. Considerations such as the nature of the signal producing system and the nature of the analytes normally determine the concentrations of the various reagents.

As mentioned above, the sample and reagents are provided in combination in the medium. While the order of addition to the medium may be varied, there will be certain preferences for some embodiments of the assay formats described herein. The simplest order of addition, of course, is to add all the materials simultaneously and determine the effect that the assay medium has on the signal as in a homogeneous assay. Alternatively, each of the reagents, or groups of reagents, can be combined sequentially. In some embodiments, an incubation step may be involved subsequent to each addition as discussed above. In heterogeneous assays, washing steps may also be employed after one or more incubation steps.

### Examination Step

In a next step of an assay method, the medium is examined for the presence of a complex comprising the analyte and antibody for the analyte. The presence and/or amount of the complex indicates the presence and/or amount of the analyte in the sample.

The phrase "measuring the amount of an analyte" refers to the quantitative, semiquantitative and qualitative determination of analyte. Methods that are quantitative, semiquantitative and qualitative, as well as all other methods for determining the analyte, are considered to be methods of measuring the amount of the analyte. For example, a method, which merely detects the presence or absence of the analyte in a sample suspected of containing the analyte, is considered to be included within the scope of the present invention. The terms "detecting" and "determining," as well as other common synonyms for measuring, are contemplated within the scope of the present invention.

In many embodiments the examination of the medium involves detection of a signal from the medium. The presence and/or amount of the signal is related to the presence and/or amount of the analyte in the sample. The particular mode of detection depends on the nature of the sps. As discussed above, there are numerous methods by which a label of an sps can produce a signal detectable by external means. Activation of a signal producing system depends on the nature of the signal producing system members.

Temperatures during measurements generally range from about 10°C to about 70°C or from about 20°C to about 45°C, or about 20°C to about 25°C. In one approach standard curves are formed using known concentrations of the analyte. Calibrators and other controls may also be used.

Luminescence or light produced from any label can be measured visually, photographically, actinometrically, spectrophotometrically, such as by using a photomultiplier or a photodiode, or by any other convenient means to determine the amount thereof, which is related to the amount of analyte in the medium. The examination for presence and/or amount of the signal also includes the detection of the signal, which is generally merely a step in which the signal is read. The signal is normally read using an instrument, the nature of which depends on the nature of the signal. The instrument may be, but is not limited to, a spectrophotometer, fluorometer, absorption spectrometer, luminometer, and chemiluminometer, for example.

### Kits comprising reagents for conducting assays

Labeled support conjugates such as, for example, labeled particle conjugates, in accordance with the principles described herein and other reagents for conducting a particular assay for an analyte may be present in a kit useful for conveniently performing an assay for the determination of an analyte. The kit may further include other reagents for performing the assay, the nature of which depend upon the particular assay format.

The reagents may each be in separate containers or various reagents can be combined in one or more containers depending on the cross-reactivity and stability of the reagents. The kit can further include other separately packaged reagents for conducting an assay such as additional sbp members, sps members, ancillary reagents, for example.

The relative amounts of the various reagents in the kits can be varied widely to provide for concentrations of the reagents that substantially optimize the reactions that need to occur during the present methods and further to optimize substantially the sensitivity of an assay. Under appropriate circumstances one or more of the reagents in the kit can be provided as a dry powder, usually lyophilized, including excipients, which on dissolution will provide for a reagent solution having the appropriate concentrations for performing a method or assay using a label particle-conjugate in accordance with the principles described herein. The kit can further include a written description of a method utilizing reagents that include a conjugate in accordance with the principles described herein.

The phrase "at least" as used herein means that the number of specified items may be equal to or greater than the number recited. The phrase "about" as used herein means that the number recited may differ by plus or minus 10%; for example, "about 5" means a range of 4.5 to 5.5.

The following discussion is directed to specific examples in accordance with the principles described herein by way of illustration and not limitation; the specific examples are not intended to limit the scope of the present disclosure and the appended claims. Numerous modifications and alternative compositions, methods, and systems may be devised without departing from the spirit and scope of the present disclosure.

### EXAMPLES

Unless otherwise indicated, materials in the experiments below may be purchased from the Sigma-Aldrich Chemical Corporation, St. Louis MO.

### Definitions:

hr = hour(s)
rpm = revolutions per minute
mg = milligram(s)
ng = nanogram(s)
pg = pictogram(s)
g = gram(s)
mL = milliliter(s)
°C = degrees Centigrade
min = minute(s)
sec = second(s)
conc = concentrated
DI = distilled water
MES = 2-(N-morpholino)ethanesulfonic acid
EDA = ethylenediamine
NHS = N-hydroxysuccinimide
MeOP = 1-methoxy-2-propanol
PEO4 = poly(ethylene oxide) with four repeating ethylene oxide units
UPA = Ultra Particle Analyzer
DTT = dithiothreitol
Me = methyl
Et = ethyl
Ac = acetate
kDa = kilodalton(s)
TLC = thin layer chromatography
Rf = retardation factor
kcount = kilocounts

### Preparation of EPRM-EDA beads:

EPRM beads (2000 mg, 20.0 mL) are added to a 40-mL vial. The EPRM beads are prepared by a procedure similar to that described in U.S. Patent No. 7,179,660 and the chemiluminescent compound is 2-(4-(N,N, di- tetradecyl)-anilino-3-phenyl thioxene with europium chelate. EDA (800 mg, 890 µL) is combined with 10 mL MES pH 6 buffer (the "Buffer") and about 4.2 mL 6N HCl. The pH of the mixture is, or is adjusted to be, about 6.9. The EDA solution is added to the EPRM beads with vortexing and the mixture is rocked at room temperature for 15 minutes. Sodium cyanoborohydride (400 mg) is combined in a 15 mL vial with 10 mL DI water and the combination is added to the bead mixture from above. The mixture is shaken at 37°C for 18-20 hours. The beads are transferred to six 40 mL centrifuge tubes. MES buffer is added to bring the volume to 35 mL and the mixture is centrifuged at 19,000 rpm for 30 min. The supernatant is decanted and the beads are re-suspended in 2 mL of the Buffer with a stir-rod and additional Buffer is added to 35 mL. The mixture is sonicated at 18 Watts power for 30 sec, using ice to keep the mixture cold. The wash/sonication step is performed 4 times to remove all activation chemical. After the last MES Buffer centrifugation, 2 mL of a buffer containing 5% MeOP and 0.1% TWEEN® 20 (the "second Buffer") is added to the tubes for the re-suspension step. Additional second buffer is added to 35 mL before sonication. The bead suspension is centrifuged at 19,000 rpm for 30 min. The supernatant is discarded. The final sonication used 12 mL of the second Buffer in each tube to give a 25 mg/mL dilution. Particle size is 277 nm as determined on a UPA instrument (Microtrac Inc., Montgomeryville PA).

### Coupling the EPRM-EDA Beads with Estriol-6-CMO NHS Ester

Activated steroid-CMO is prepared by adding to a 2 mL vial 5.0 mg estriol-6-CMO, EDAC (12 mg) and NHS (16 mg) plus 1.25 mL dry acetonitrile. The vial contents are vortexed vigorously. The mixture is allowed to stir at room temperature for 120 min. TLC (EtOAc:EtOH =3:1) indicates no starting steroid material. Rf (NHS ester) = 0.8; Rf (6-Ketoestriol-6-CMO) = 0.05. The activated steroid-CMO is coupled to EPRM-EDA beads by adding, to a 25-mL vial dropwise with stirring, 16 mL (400 mg) of bead suspension from above, 4.32 mg (1.08 mL) of activated estriol-CMO from above. After the completion of addition, the mixture is stirred for 20 hours at room temperature. The resultant beads are washed by transferring the above mixture to a 40-mL centrifuge tube. The second Buffer is added to bring tubes to 35 mL. Centrifugation is carried out at 19,000 rpm for 30 min. The supernatant is decanted and the beads are re-suspended in 10 mL of the second Buffer with a stir-rod. Additional second Buffer is added to 35 mL. The tubes are subjected to sonication at 18 Watts power for 60 sec using ice to keep the tubes cold. The beads are centrifuged as above and washed three additional times with the second Buffer. The beads are re-suspended in Hapten Wash Buffer (50 mM HEPES, 300 mM NaCl, 1 mM EDTA, 0.01% Neomycin Sulfate, 0.1% TRITON® 405X and 0.15% PROCLIN® 300, pH 7.2) for 2 more washes. The final re-suspension uses 10 mL to give 10 mg/mL suspension and is sonicated for 30 sec. Particle size of about 275 nm is determined on the UPA instrument. The resultant beads are designated EPRM-EDA-Estradiol beads.

### Assay for Estriol Analyte

Assays were carried out on a DIMENSION® VISTA® analyzer (Siemens Healthcare Diagnostics Inc., Deerfield, IL) following the protocol of the manufacturer for an induced luminescence assay and using sample solutions containing varying amounts of estradiol. In this example, the assay uses, as a chemiluminescent reagent, a label particle-conjugate in accordance with the principles described herein where the label of the particle-conjugate is a chemiluminescent compound. The photosensitizer reagent comprises an antibody for the analyte by virtue of the binding of streptavidin sensitizer particle with biotinylated antibody. The analyte competes with the particle-conjugate for binding to the antibody for the analyte. If the analyte is present, the fewer is the number of molecules of labeled particle-conjugate that come into close proximity with the photosensitizer reagent. Therefore, there will be a decrease in the assay signal. The photosensitizer generates singlet oxygen and activates the chemiluminescent compound when the two labels are in close proximity. The activated chemiluminescent compound subsequently produces light. The amount of light produced is related to the amount of the complex formed, which in turn is related to the amount of analyte present in the sample.

The streptavidin-sensitizer bead ("sensibead") is prepared using a method analogous to that described in U.S. Patent Nos. 6,153,442, 7.022,529, 7,229,842 and U.S. Patent Application Publication No. 20050118727A. The photosensitizer was bis-(trihexyl)-silicon-t-butyl-phthalocyanine. The EPRM-EDA-Estriol prepared as described above was employed as a "chemibead reagent." The biotinylated antibody reagent is prepared using monoclonal antibody specific for estriol (sheep monoclonal from Bioventix, Farnham, Surrey, UK) and biotinylating amine groups of the antibody by reaction with NHS-PEO4-biotin (Pierce Chemical Company, Rockford IL) in a manner similar to that described in U.S. Patent Application Publication No. 2009/0258435A1, the relevant portions of which are incorporated herein by reference.

At time t = zero sec, 20 µL biotinylated antibody reagent and 20 µL water were added to a reaction vessel. Sample, 12 µL, was added 21.6 seconds later, followed by 8 µL water. At t = 414.0 seconds, 40 µL chemibead reagent was added followed by 20 µL of water. Sensibead reagent was then dispensed at 457.2 seconds. Measurements were taken 601.2 seconds after initiation of the reaction sequence.

The results are summarized in Table 1 below.

**Table 1**

| Estriol (pg/mL) | kcount |
|---|---|
| 0.00 | 1491 |
| 50.5 | 1136 |
| 320.6 | 424 |
| 652.8 | 213 |
| 1585.5 | 84 |

The above experiments are repeated using a procedure similar to that described above and using, as the hydrophobic hapten, progesterone, testosterone, and vitamin D. The results for progesterone are summarized in Table 2.

**Table 2**

| Progesterone (ng/mL) | kcount |
|---|---|
| 0.00 | 676 |
| 0.97 | 487 |
| 8.97 | 108 |
| 17.2 | 53 |
| 39.4 | 22 |

## Claims

1. A composition comprising a water insoluble support having on a surface thereof at least an inner aminodextran layer and an outer dextran aldehyde layer wherein the outer dextran_aldehyde layer has pendant moieties each comprising a terminal amine group wherein a point of linkage of each of the pendant moieties to the dextran aldehyde layer_comprises a secondary amine linkage, but does not comprise an amide bond, and wherein each of the pendant moieties comprises an alkylene of about 2 to about 10 carbon atoms or a poly(alkylene ether) of about 2 to about 20 alternating ether linkages and alkylene moieties .

2. The composition according to claim 1 wherein the support is a particle.

3. The composition according to claim 1 wherein the pendant moiety comprises an alkylene of about 2 to about 5 carbon atoms or a poly(alkylene ether) of about 2 to about 5 alternating ether linkages and alkylene moieties.

4. A method of conjugating a member of a specific binding pair to a support, the method comprising reacting the terminal amine group of the composition of claim 1 with the member of the specific binding pair that comprises an amine-reactive functionality.

5. The method according to claim 4 wherein the member of the specific binding pair is a hapten.

6. The method of claim 5 wherein the hapten is a hormone.

7. A method of forming a conjugate of a hapten and the composition of claim 1, the method comprising forming a linkage between the hapten functionalized with an amine-reactive functionality and a terminal amino group of a pendant moiety of the composition.

8. A method of forming a conjugate of a steroid hormone and a water insoluble particle wherein the particle comprises an inner aminodextran layer and an outer dextran aldehyde layer, the method comprising: forming a composition of claim 1, and reacting the terminal amine group of the pendant moiety with the steroid hormone comprising an amine-reactive functionality to form the conjugate of the steroid hormone and the particle.

9. The method according to claim 8 wherein the particles are latex particles, or wherein the particles comprise a label, wherein the label is preferably selected from the group consisting of chemiluminescent compositions and sensitizer compositions, or wherein the steroid hormone is selected from the group consisting of progestogens, estrogens, androgens, glucosteroids, mineralocorticoids, and secosteroids.

10. The method according to claim 8 wherein the amine reactive functionality of the steroid hormone is a functionalized ketone, a functionalized aldehyde, or a functionalized alcohl.

11. The method according to claim 8 wherein the amine reactive functionality of the steroid hormone is carboxymethoxylamine-functionalized.

## Patentansprüche

1. Zusammensetzung, umfassend einen wasserunlöslichen Träger, der auf einer Oberfläche davon mindestens eine innere Aminodextranschicht und eine äußere Dextranaldehydschicht aufweist, wobei die äußere Dextranaldehydschicht Seitengruppen aufweist, die jeweils eine endständige Amingruppe umfassen, wobei ein Verknüpfungspunkt jeder der Seitengruppen an der Dextranaldehydschicht eine sekundäre Aminverknüpfung umfasst, aber keine Amidbindung umfasst, und wobei jede der Seitengruppen ein Alkylen mit etwa 2 bis etwa 10 Kohlenstoffatomen oder einen Poly(alkylenether) von etwa 2 bis etwa 20 umfasst, bei denen die Etherverknüpfungen und Alkylenreste alternieren.

2. Zusammensetzung nach Anspruch 1, wobei der Träger ein Teilchen ist.

3. Zusammensetzung nach Anspruch 1, wobei die Seitengruppe ein Alkylen mit etwa 2 bis etwa 5 Kohlenstoffatomen oder einen Poly(alkylenether) von etwa 2 bis etwa 5 alternierenden Etherverknüpfungen und Alkylenresten umfasst.

4. Verfahren zur Konjugation eines Elements eines spezifischen Bindungspaares an einen Träger, wobei das Verfahren die Umsetzung der endständigen Amingruppe der Zusammensetzung nach Anspruch 1 mit dem Element des spezifischen Bindungspaares umfasst, das eine aminreaktive Funktionalität umfasst.

5. Verfahren nach Anspruch 4, wobei das Element des spezifischen Bindungspaares ein Hapten ist.

6. Verfahren nach Anspruch 5, wobei das Hapten ein Hormon ist.

7. Verfahren zur Bildung eines Konjugats eines Haptens und der Zusammensetzung nach Anspruch 1, wobei das Verfahren das Bilden einer Verknüpfung zwischen dem Hapten, das mit einer aminreaktiven Funktionalität funktionalisiert ist, und einer endständigen Aminogruppe einer Seitengruppe der Zusammensetzung umfasst.

8. Verfahren zur Bildung eines Konjugats eines Steroidhormons und eines wasserunlöslichen Teilchens, wobei das Teilchen eine innere Aminodextranschicht und eine äußere Dextranaldehydschicht umfasst, wobei das Verfahren umfasst: Bilden einer Zusammensetzung nach Anspruch 1 und Umsetzen der endständigen Amingruppe der Seitengruppe mit dem Steroidhormon, das eine aminreaktive Funktionalität umfasst, um das Konjugat des Steroidhormons und des Teilchens zu bilden.

9. Verfahren nach Anspruch 8, wobei die Teilchen Latexteilchen sind oder wobei die Teilchen eine Markierung umfassen, wobei die Markierung vorzugsweise ausgewählt ist aus der Gruppe, bestehend aus chemilumineszierenden Zusammensetzungen und Sensibilisatorzusammensetzungen, oder wobei das Steroidhormon ausgewählt ist aus der Gruppe, bestehend aus Progestogenen, Östrogenen, Androgenen, Glucosteroiden, Mineralocorticoiden und Secosteroiden.

10. Verfahren nach Anspruch 8, wobei die aminreaktive Funktionalität des Steroidhormons ein funktionalisiertes Keton, ein funktionalisiertes Aldehyd oder ein funktionalisierter Alkohol ist.

11. Verfahren nach Anspruch 8, wobei die aminreaktive Funktionalität des Steroidhormons carboxymethoxylaminfunktionalisiert ist.

## Revendications

1. Composition comprenant un support insoluble dans l'eau présentant sur une surface de celui-ci au moins une couche d'aminodextrane interne et une couche de dextran-aldhéyde externe dans laquelle la couche de dextran-aldéhyde externe présente des fractions pendantes comprenant chacune un groupe amine terminal dans laquelle un point de liaison de chacune des fractions pendantes de la couche de dextran-aldéhyde comprend une liaison amine secondaire, mais ne comprend pas une liaison amide, et dans laquelle chacune des fractions pendantes comprend un alkylène d'environ 2 à environ 10 atomes de carbone ou un poly(alkylène éther) d'environ 2 à environ 20 liaisons éther et fractions d'alkylène alternées.

2. Composition selon la revendication 1 dans laquelle le support est une particule.

3. Composition selon la revendication 1 dans laquelle la fraction pendante comprend un alkylène d'environ 2 à environ 5 atomes de carbone ou un poly(alkylène éther) d'environ 2 à environ 5 liaisons éther et fractions d'alkylène alternées.

4. Procédé de conjugaison d'un élément d'une paire de liaison spécifique à un support, le procédé comprenant une mise en réaction du groupe amine terminal de la composition selon la revendication 1 avec l'élément de la paire de liaison spécifique qui comprend une fonction réactive amine.

5. Procédé selon la revendication 4 dans lequel l'élément de la paire de liaison spécifique est un haptène.

6. Procédé selon la revendication 5 dans lequel l'haptène est une hormone.

7. Procédé de formation d'un conjugué d'un haptène et de la composition de la revendication 1, le procédé comprenant une formation d'une liaison entre l'haptène fonctionnalisé avec une fonction réactive amine et un groupe amine terminal d'une fraction pendante de la composition.

8. Procédé de formation d'un conjugué d'une hormone stéroïde et d'une particule insoluble dans l'eau dans lequel la particule comprend une couche d'aminodextrane interne et une couche de dextran-aldéhyde externe, le procédé comprenant : une formation d'une composition selon la revendication 1, et une mise en réaction du groupe amine terminal de la fraction pendante avec l'hormone stéroïde comprenant une fonction réactive amine pour former le conjugué de l'hormone stéroïde et la particule.

9. Procédé selon la revendication 8 dans lequel les particules sont des particules de latex, ou dans lequel les particules comprennent un marqueur, dans lequel le marqueur est de préférence choisi parmi le groupe constitué de compositions chimioluminescentes et de compositions de sensibilisateur, ou dans lequel l'hormone stéroïde est choisie parmi le groupe constitué de progestagènes, d'oestrogènes, d'androgènes, de glucostéroïdes, de minéralocorticoïdes, et de sécostéroïdes.

10. Procédé selon la revendication 8 dans lequel la fonction réactive amine de l'hormone stéroïde est une cétone fonctionnalisée, un aldéhyde fonctionnalisé, ou un alcool fonctionnalisé.

11. Procédé selon la revendication 8 dans lequel la fonction réactive amine de l'hormone stéroïde est fonctionnalisée à la carboxyméthoxylamine.
